# EUROPÄISCHE PATENTANMELDUNG

(11) **EP 0 979 872 A1**
(43) Veröffentlichungstag der Anmeldung: **16.02.2000**
(21) Anmeldenummer: 99115140.8
(22) Anmeldetag: 11.08.1999
(51) Int. Cl.: C12N 15/12, C07K 14/72, A01K 67/027

(54) **Tiermodell zur Entstehung und Therapie von Diabetes Mellitus**

(30) Priorität: 11.08.1998 DE 19836382
(71) Anmelder: GSF-Forschungszentrum für Umwelt und Gesundheit GmbH, 85764 Oberschleissheim (DE); Wolf, Eckhard, Prof. Dr., 80339 München (DE)
(72) Erfinder: Peters, Heiko, 85764 Oberschleissheim (DE); Balling, Rudolf, 81549 München (DE); Volz, Anja, 85764 Oberschleissheim (DE); Göke, Burkhard, 35037 Marburg (DE); Wolf, Eckhard, 80339 München (DE)
(74) Vertreter: Reinhard - Skuhra - Weise & Partner

(57) **Zusammenfassung**

Die Erfindung betrifft eine rekombinante DNA, umfassend einen Promotor, der in den ß-Zellen des endokrinen Pankreas exprimierbar ist, eine Sequenz, die für ein GIP-Rezeptorprotein oder ein aktives Fragment hiervon kodiert, das befähigt ist, das Peptidhormon GIP zu binden, ohne eine Signaltransduktion zu initiieren, eine Terminationssequenz und eine Polyadenylierungssequenz, wobei die DNA bei Expression in einem geeigneten Wirt einen Diabetes-Phänotyp bewirkt sowie transgene, nicht menschliche Säugetiere, die diese rekombinante DNA enthalten.

SEQUENZPROTOKOLL

<110> GSF-Forschungszentrum f. Umwelt und Gesundheit
      Wolf, Prof. Dr. Eckhard

<120> Neues Tiermodell zur Entstehung und Therapie von
      Diabetes Mellitus

<130> P11539

<141> 1999-08-11

<150> DE 198 36 382.6
<151> 1998-08-11

<160> 3

<170> PatentIn Ver. 2.1

<210> 1
<211> 466
<212> PRT
<213> Homo sapiens

<400> 1
Met Thr Thr Ser Pro Ile Leu Gln Leu Leu Leu Glu Leu Ser Leu Cys
  1               5                  10                  15

Gly Leu Leu Leu Gln Arg Ala Glu Thr Gln Ser Lys Gly Gln Thr Ala
             20                  25                  30

Gly Glu Leu Tyr Gln Arg Trp Glu Arg Tyr Arg Arg Glu Cys Gln Glu
         35                  40                  45

Tyr Leu Ala Ala Ala Glu Pro Pro Ser Gly Leu Ala Cys Asn Gly Ser
     50                  55                  60

Phe Asp Met Tyr Val Cys Trp Asp Tyr Ala Ala Pro Asn Ala Tyr Ala
 65                  70                  75                  80

Arg Ala Ser Cys Pro Trp Tyr Leu Pro Trp His His His Val Ala Ala
                 85                  90                  95

Gly Phe Val Leu Arg Gln Cys Arg Ser Asp Gly Gln Trp Gly Leu Trp
            100                 105                 110

Arg Asp His Thr Gln Cys Glu Asn Pro Glu Lys Asn Glu Ala Phe Leu
        115                 120                 125

Asp Gln Arg Leu Ile Leu Glu Arg Leu Gln Val Met Tyr Thr Val Gly
    130                 135                 140

Tyr Ser Leu Ser Leu Ala Thr Leu Leu Leu Ala Leu Leu Ile Leu Ser
145                 150                 155                 160

Leu Phe Arg Arg Leu His Cys Thr Arg Asn Tyr Ile His Ile Asn Leu
                165                 170                 175

Phe Thr Ser Phe Met Leu Arg Ala Ala Ala Ile Leu Ser Arg Asp Arg
            180                 185                 190

Leu Leu Pro Arg Pro Gly Pro Tyr Leu Gly Asp Gln Ala Leu Ala Leu
        195                 200                 205

Trp Asn Gln Ala Leu Ala Ala Cys Arg Thr Ala Gln Ile Val Thr Gln
    210                 215                 220

Tyr Cys Val Gly Ala Asn Tyr Thr Trp Leu Leu Val Glu Gly Val Tyr
225                 230                 235                 240

Leu His Ser Leu Leu Val Leu Val Gly Gly Ser Glu Glu Gly His Phe
                245                 250                 255

Arg Tyr Tyr Leu Leu Leu Gly Trp Gly Ala Pro Ala Leu Phe Val Ile
            260                 265                 270

Pro Trp Val Ile Val Arg Tyr Leu Tyr Glu Asn Thr Gln Cys Trp Glu
        275                 280                 285

Arg Asn Glu Val Lys Ala Ile Trp Trp Ile Ile Arg Thr Pro Ile Leu
    290                 295                 300

Met Thr Ile Leu Ile Asn Phe Leu Ile Phe Ile Arg Ile Leu Gly Ile
305                 310                 315                 320

Leu Leu Ser Lys Leu Arg Thr Arg Gln Met Arg Cys Arg Asp Tyr Arg
                325                 330                 335

Val Arg Leu Ala Arg Ser Thr Leu Thr Leu Val Pro Leu Leu Gly Val
            340                 345                 350

His Glu Val Val Phe Ala Pro Val Thr Glu Glu Gln Ala Arg Gly Ala
        355                 360                 365

Leu Arg Phe Ala Lys Leu Gly Phe Glu Ile Phe Leu Ser Ser Phe Gln
    370                 375                 380

Gly Phe Leu Val Ser Val Leu Tyr Cys Phe Ile Asn Lys Glu Val Gln
385                 390                 395                 400

Ser Glu Ile Arg Arg Gly Trp His His Cys Arg Leu Arg Arg Ser Leu
                405                 410                 415

Gly Glu Glu Gln Arg Gln Leu Pro Glu Arg Leu Phe Arg Ala Leu Pro
            420                 425                 430

Ser Gly Ser Gly Pro Gly Glu Val Pro Thr Ser Arg Gly Leu Ser Ser
        435                 440                 445

Gly Thr Leu Pro Gly Pro Gly Asn Glu Ala Ser Arg Glu Leu Glu Ser
    450                 455                 460

Tyr Cys
465

<210> 2
<211> 23
<212> PRT
<213> Homo sapiens

<400> 2
Gly Ile Leu Leu Ser Lys Leu Arg Thr Arg Gln Met Arg Cys Arg Asp
  1               5                  10                  15

Tyr Arg Val Arg Leu Ala Arg
            20

<210> 3
<211> 458
<212> PRT
<213> Homo sapiens

<400> 3
Met Thr Thr Ser Pro Ile Leu Gln Leu Leu Leu Glu Leu Ser Leu Cys
1               5                   10                  15

Gly Leu Leu Leu Gln Arg Ala Glu Thr Gln Ser Lys Gly Gln Thr Ala
            20                  25                  30

Gly Glu Leu Tyr Gln Arg Trp Glu Arg Tyr Arg Arg Glu Cys Gln Glu
        35                  40                  45

Tyr Leu Ala Ala Ala Glu Pro Pro Ser Gly Leu Ala Cys Asn Gly Ser
    50                  55                  60

Phe Asp Met Tyr Val Cys Trp Asp Tyr Ala Ala Pro Asn Ala Tyr Ala
65                  70                  75                  80

Arg Ala Ser Cys Pro Trp Tyr Leu Pro Trp His His His Val Ala Ala
                85                  90                  95

Gly Phe Val Leu Arg Gln Cys Arg Ser Asp Gly Gln Trp Gly Leu Trp
            100                 105                 110

Arg Asp His Thr Gln Cys Glu Asn Pro Glu Lys Asn Glu Ala Phe Leu
        115                 120                 125

Asp Gln Arg Leu Ile Leu Glu Arg Leu Gln Val Met Tyr Thr Val Gly
    130                 135                 140

Tyr Ser Leu Ser Leu Ala Thr Leu Leu Leu Ala Leu Leu Ile Leu Ser
145                 150                 155                 160

Leu Phe Arg Arg Leu His Cys Thr Arg Asn Tyr Ile His Ile Asn Leu
                165                 170                 175

Phe Thr Ser Phe Met Leu Arg Ala Ala Ala Ile Leu Ser Arg Asp Arg
            180                 185                 190

Leu Leu Pro Arg Pro Gly Pro Tyr Leu Gly Asp Gln Ala Leu Ala Leu
        195                 200                 205

Trp Asn Gln Ala Leu Ala Ala Cys Arg Thr Ala Gln Ile Val Thr Gln
    210                 215                 220

Tyr Cys Val Gly Ala Asn Tyr Thr Trp Leu Leu Val Glu Gly Val Tyr
225                 230                 235                 240

Leu His Ser Leu Leu Val Leu Val Gly Gly Ser Glu Glu Gly His Phe
                245                 250                 255

Arg Tyr Tyr Leu Leu Leu Gly Trp Gly Ala Pro Ala Leu Phe Val Ile
            260                 265                 270

Pro Trp Val Ile Val Arg Tyr Leu Tyr Glu Asn Thr Gln Cys Trp Glu

        275                 280                 285

Arg Asn Glu Val Lys Ala Ile Trp Trp Ile Ile Arg Thr Pro Ile Leu
    290                 295                 300

Met Thr Ile Leu Ile Asn Phe Leu Ile Phe Ile Arg Ile Leu Thr Arg
305                 310                 315                 320

Gln Met Arg Cys Arg Asp Tyr Arg Val Arg Leu Glu Arg Ser Thr Leu
                325                 330                 335

Thr Leu Val Pro Leu Leu Gly Val His Glu Val Val Phe Ala Pro Val
            340                 345                 350

Thr Glu Glu Gln Ala Arg Gly Ala Leu Arg Phe Ala Lys Leu Gly Phe
        355                 360                 365

Glu Ile Phe Leu Ser Ser Phe Gln Gly Phe Leu Val Ser Val Leu Tyr
    370                 375                 380

Cys Phe Ile Asn Lys Glu Val Gln Ser Glu Ile Arg Arg Gly Trp His
385                 390                 395                 400

His Cys Arg Leu Arg Arg Ser Leu Gly Glu Glu Gln Arg Gln Leu Pro
                405                 410                 415

Glu Arg Leu Phe Arg Ala Leu Pro Ser Gly Ser Gly Pro Gly Glu Val
            420                 425                 430

Pro Thr Ser Arg Gly Leu Ser Ser Gly Thr Leu Pro Gly Pro Gly Asn
        435                 440                 445

Glu Ala Ser Arg Glu Leu Glu Ser Tyr Cys
    450                 455

## Beschreibung

Die vorliegende Erfindung betrifft ein nicht humanes, transgenes Säugetier, das sich als Tiermodell zur Erforschung von Diabetes und zur Entwicklung neuer Therapien von Diabetes eignet und rekombinante DNA, die zur Herstellung des transgenen, nicht humanen Säugetiers eingesetzt wird, diese rekombinante DNA enthaltende Vektoren und Zellinien sowie ein Verfahren zur Herstellung des transgenen, nicht humanen Säugetiers.

### Diabetes

Die Aufrechterhaltung einer konstanten Glukosekonzentration des Blutes ist für die Vitalität des menschlichen Organismus von essentieller Bedeutung. Sie wird durch fein abgestimmte Regulationsmechanismen gesteuert, wobei der durch Insulin vermittelten Glukoseaufnahme der Körperzellen eine zentrale Rolle zukommt. Diabetes entsteht, wenn die Bauchspeicheldrüse entweder kein bzw. zuwenig Insulin produziert, oder die durch Insulin vermittelte Glukoseaufnahme der Körperzellen gestört ist. Diabetes mellitus ist als die häufigste vererbbare Stoffwechselkrankheit ein großes medizinisches und volkswirtschaftliches Problem. Über 200 Millionen Menschen sind weltweit an Diabetes mellitus Typ II erkrankt. Die Morbidität des Diabetes mellitus beträgt 2-3%.

Über die molekularen Mechanismen der Entstehung von Diabetes ist bislang wenig bekannt. Man geht davon aus, daß die Ursachen polyfaktoriell sind und neben genetischen Veränderungen auch Eß- und Lebensgewohnheiten umfassen. Gegenwärtig werden in der medizinischen Grundlagenforschung große Anstrengungen unternommen, die molekularen Vorgänge bei der Entstehung von Diabetes zu verstehen.

Eine ideale Vorausetzung für die erfolgreiche Entwicklung von Verfahren zur Diagnose, Behandlung und Prävention von Diabetes beim Menschen ist die Verfügbarkeit von Tiermodellen, in denen einzelne Komponenten aus der gesamten Pathologie der Entstehung von Diabetes gezielt untersucht werden können. Kann beispielsweise im Tiermodell gezeigt werden, daß die funktionelle Inaktivierung eines einzigen Proteins zur Entstehung von Diabetes führen kann, müssen Veränderungen (Mutationen, Aktivitätsverlust, Aktivitätsveränderungen etc.) dieses Proteins auch bei der Entstehung von Diabetes des Menschen in Betracht gezogen werden. Die Identifizierung eines pathogenen Mechanismus für die Diabetes-Entstehung im Tiermodell schafft somit die Voraussetzung für die Entwicklung neuer Therapeutika. Darüber hinaus kann dieses Tiermodell in idealer Weise als Testsystem zur Überprüfung bereits angewendeter, aber auch neu entwickelter Therapeutika integriert werden.

### Die Funktion von GIP

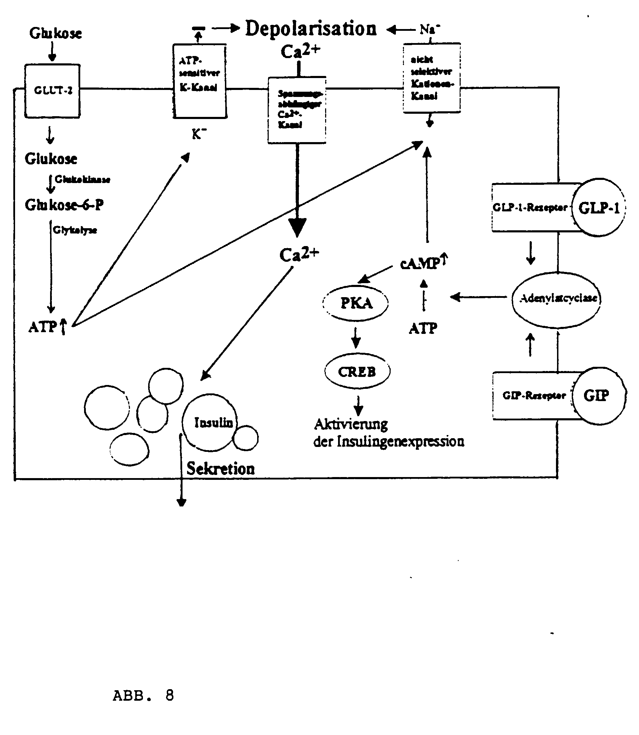

GIP (Glucose-dependent insulinotropic (insulin-releasing) polypeptide; ältere Bezeichnung: gastric inhibitory peptide) ist ein Peptidhormon, das nach oraler Glukoseaufnahme von endokrinen Darmzellen freigesetzt wird und die Glukose-induzierte Insulinsekretion und -produktion verstärkt. GIP gelangt nach Nahrungsaufnahme über das Blut zu den Beta-Zellen des endokrinen Pankreas, wo es vom GIP-Rezeptor gebunden wird. Der GIP-Rezeptor leitet nach der Bindung des Hormons eine Signalkaskade in der Beta-Zelle ein, die zu einem Anstieg der intrazellulären cAMP-Konzentration führt (cAMP: cyklisches Adenosinrmonophosphat). cAMP ist ein sekundärer Botenstoff, der über weitere Zwischenschritte schließlich in den Beta-Zellen des endokrinen Pankreas sowohl die Freisetzung von Insulin als auch die Transkription des Insulin-Gens aktiviert (Abb. 8).

Obwohl eine Reihe von Tiermodellen für Diabetes entwickelt wurden (db/db mouse: Zask et al., 1990; Roe et al., 1994; ob/ob mouse: Gettys et al., 1995; NZO-mouse: Andrikopoulos et al., 1993; NSY-mouse: Ueda et al., 1995), bieten diese Modelle bislang nicht die Möglichkeit, die Rolle eines defekten GIP-Rezeptors bei der Entstehung von Diabetes zu klären.

Klinische Studien haben gezeigt, daß die Verabreichung von GLP-1 (Glucagon like peptide) bei Patienten mit Diabetes mellitus Typ II die Insulinfreisetzung teilweise wiederherstellen kann. Interessanterweise sind bei diesen Versuchen dagegen externe Gaben von GIP wirkungslos. Neben der Tatsache, daß Typ II Diabetiker normale oder sogar erhöhte GIP-Plasmaspiegel aufweisen (Fehmann et al., 1995), legen diese Befunde die Annahme nahe, daß Mutationen bzw. Aktivitätsverluste des GIP-Rezeptors eine mögliche Ursache für die Entstehung von Diabetes mellitus Typ II sind. Ein funktioneller Zusammenhang zwischen einem veränderten GIP-Rezeptor und der Entwicklung von Diabetes konnte jedoch bislang nicht gezeigt werden.

### Die Rolle des GIP-Rezeptors

Der GIP-Rezeptor gehört zu einer Familie von ZelloberflächenRezeptoren, die als G-Protein gekoppelte Rezeptoren bezeichnet werden. Alle Mitglieder dieser Familie sind in der Zellmembran verankert und strukturell ähnlich aufgebaut: Sie besitzen außerhalb der Zelle Proteindomänen, die für die Bindung des Hormons wichtig sind. Die Zellmembran wird durch sieben Domänen des Rezeptors durchzogen, die jeweils durch kurze Proteinschleifen voneinander getrennt sind. Von diesen Proteinschleifen ist u.a. die dritte intrazelluläre Schleife von besonderer Bedeutung, da bei verwandten Rezeptoren bereits gezeigt werden konnte, daß an diese Schleife G-Proteine binden können. G-Proteine sind heterodimere Proteine, die in ihrer inaktiven Form das Nukleotid GDP (Guanosindiphosphat) binden und mit den G-Protein gekoppelten Rezeptoren assoziiert sind. Die Stimulation des Rezeptors durch seinen Ligand führt zu einer Konformationsänderung des gebundenen G-Proteins, wodurch die Affinität des G-Proteins zu GDP herabgesetzt wird und dieses durch GTP (Guanosintriphosphat) ausgetauscht wird. Daraufhin dissoziiert eine der drei Untereinheiten des G-Proteins ab und aktiviert bzw. inhibiert verschiedene Effektormoleküle, wie beispielsweise die Adenylatcyclase der Beta-Zelle.

Die cDNAs des GIP-Rezeptorgens sind inzwischen von der Ratte, vom Hamster, und vom Menschen isoliert und sequenziert worden. Ihre abgeleiteten Aminosäuresequenzen zeigen zueinander ca. 80% Homologie. Der humane GIP-Rezeptor bindet GIP mit einer Affinität von Kd=1,13 X 10-8 M und zeigt keine Bindung zu verwandten Peptiden (Volz et al. 1995). Zur funktionellen Charakterisierung des humanen GIP-Rezeptors wurde die cDNA durch Transfektion in CHL-Zellen zur Expression gebracht. Nach Stimulation der transtizierten Zellen mit GIP konnte gezeigt werden, daß der Spiegel von cAMP, das die Signaltransduktion vermittelt, erhöht ist (Volz et al., 1995).

Die cDNA des GIP-Rezeptors des Menschen diente als Grundlage der Experimente, deren Ergebnisse im folgenden beschrieben werden.

Aufgabe der vorliegenden Erfindung ist die Bereitstellung eines neuen Tiermodells, um die Entstehung, Therapie und die Spätfolgen von Diabetes mellitus, insbesondere Typ II, zu untersuchen.

Diese Aufgabe wird erfindungsgemäß dadurch gelöst, daß eine rekombinante DNA bereitgestellt wird, umfassend einen Promotor, der in den ß-Zellen des endokrinen Pankreas exprimierbar ist, eine Sequenz, die für ein GIP-Rezeptorprotein oder ein aktives Fragment hiervon kodiert, das befähigt ist, das Peptidhormon GIP zu binden, ohne eine Signaltransduktion zu initiieren, eine Terminationssequenz und eine Polyadenylierungssequenz, wobei die DNA bei Expression in einem geeigneten Wirt einen Diabetes-Phänotyp bewirkt.

Erfindungsgemäß konnte zum ersten Mal gezeigt werden, daß es möglich ist, ein GIP-Rezeptor-Protein oder ein aktives Fragment hiervon zu schaffen, das zwar das Peptidhormon GIP zu binden vermag, jedoch keine Signaltransduktion mehr initiieren kann. Dies wird dadurch erreicht, daß in das GIP-Rezeptorprotein, das aktive Fragment hiervon bzw. in die hierfür kodierende DNA eine oder mehrere Deletionen, Additionen und/oder eine oder mehrere Basenaustausche eingeführt werden, so daß durch diese Mutation(en) zwar eine Bindung von GIP ermöglicht wird, die hierdurch üblicherweise initiierte Signaltransduktion (Anstieg der cAMP-Konzentration) jedoch nicht mehr stattfindet.

Die Beibehaltung der Bindungsfähigkeit von GIP an das GIP-Rezepter-Protein bei gleichzeitig fehlender Signalweiterleitung wurde erfindungsgemäß durch Einführen von Mutationen in die dritte intrazelluläre Schleife, die vermutlich an der G-Protein-Bindung beteiligt ist, erreicht. Hierzu können bevorzugt die Aminosäuren 321 bis 328 deletiert und die Aminosäure in der Position 342 ausgetauscht werden, wobei hier bevorzugt ein Austausch des Alanins durch Glutaminsäure stattfindet. Dies ist jedoch nur eine Möglichkeit, um das erfindungsgemäße GIP-Rezeptor-Protein oder ein aktives Fragment hiervon mit der Fähigkeit zur Bindung des GIP-Peptidhormons ohne Initiation der Signaltransduktion zu schaffen. Es können auch andere Mutationen, z.B. Deletionen, Insertionen, Additionen und/oder Basenaustausche durchgeführt werden, die zum gewünschten Ergebnis führen. Der Durchschnittsfachmann wird dies, ausgehend von der vorliegenden Erfindung, im Rahmen seines Fachwissens und Fachkönnens durchführen können.

Die oben angegebenen Sequenzpositionen beziehen sich auf die in Volz et al. veröffentlichte Sequenz des GIP-Rezeptors. Auf diese Veröffentlichung wird hier vollinhaltlich Bezug genommen.

Der erfindungsgemäß in der rekombinanten DNA eingesetzte Promotor ist ein Insulin-Promotor. Selbstverständlich können auch andere, dem Fachmann zur Verfügung stehende Promotoren eingesetzt werden, die geeignet sind, die GIP-Rezeptor-Proteinsequenz oder ein aktives Fragment hiervon in den ß-Zellen des endokrinen Pankreas zu exprimieren. Bei der für das GIP-Rezeptor-Protein oder ein aktives Fragment hiervon kodierenden Sequenz handelt es sich entweder um eine genomische DNA oder eine cDNA aus einem beliebigen Säugerorganismus, bevorzugt aus Menschen.

In der vorliegenden Ausführungsform wurde die für das GIP-Rezeptor-Protein kodierende Sequenz in den RIP1-Vektor kloniert, der in der Veröffentlichung von Hanahan beschrieben ist. Auf diese Veröffentlichung wird hier vollinhaltlich Bezug genommen. Es können auch andere Vektoren eingesetzt werden, die eine zellspezifische Expression des Rezeptorproteins in den ß-Zellen des Pankreas erlauben.

Von der Erfindung umfaßt werden auch Vektoren, die die rekombinante DNA der Erfindung enthalten, eukaryontische oder prokaryontische Zellinien, enthaltend eine rekombinante DNA oder einen Vektor der vorliegenden Erfindung sowie das rekombinante GIP-Rezeptor-Protein oder ein aktives Fragment hiervon und die hierfür kodierende DNA, die so abgeändert wurden, daß das Peptidhormon GIP zu binden vermag, das vom Hormon ausgelöste Signal jedoch nicht mehr weitergeleitet wird.

Von der Erfindung umfaßt werden auch transgene, nicht menschliche Säugetiere, die die oben beschriebene rekombinante DNA enthalten und das rekombinante GIP-Rezeptor-Protein exprimieren sowie das rekombinante GIP-Rezeptor-Protein.

Die erfindungsgemäß erzeugten transgenen, nicht menschlichen Säugetiere dienen als Modellsystem zur Untersuchung der Entstehung, Therapie und Spätfolgen von Diabetes mellitus, insbesondere Diabetes mellitus Typ II.

Nachfolgend wird die Erfindung anhand von Abbildungen und von Ausführungsbeispielen näher beschrieben. Die Erfindung ist jedoch nicht auf das vorliegende Beispiel beschränkt. Im Rahmen der vorliegenden Patentansprüche kann das konkrete Ausführungsbeispiel abgeändert werden, ohne von der Erfindung abzuweichen.

Die anliegenden Abbildungen zeigen:
- Abbildung 1:: Vereinfachte Darstellung der Aminosäuresequenz des humanen GIP-Rezeptors und dessen Anordnung in der Plasmamembran.
- Abbildung 2:: Bindung von GIP nach Transfektion des mutierten GIP-Rezeptors in CHL-Zellen (Abb. 2A); Abwesenheit der Signaltransduktion nach Stimulation der so veränderten CHL-Zellen mit GIP (vergl. Abb. 2B).
- Abbildung 3:: Struktur des RIP1-Vektors
- Abblidung 4:: Genotypisierungen von potentiell transgenen Mäusen.
- Abbildung 5:: Entwicklung der Glukosurie in den transgenen Mauslinien GIPR^{dn}-A und GIPR^{dn}-B.
- Abbildung 6:: Erhöhte Glukosekonzentration im Blut der transgenen Mauslinien GIPR^{dn}-A und GIPR^{dn}-B.
- Abbildung 7:: Gestörte Glukosetoleranz in männlichen Mäusen der transgenen Mauslinie GIPR^{dn}-C.
- Abbildung 8:: Vereinfachte Darstellung der Aktivierung der Insulinsekretion und der Transkription des Insulingens durch die Wirkung der Peptidhormone GIP und GLP-1.

Die vorliegende Erfindung stellt somit ein neues, transgenes, nicht humanes Säugetier (bevorzugt Maus) bereit, das als Folge einer pankreasspezifischen Expression eines funktionell inaktiven GIP-Rezeptors in den ersten sechs Lebensmonaten eine schwere Form von Diabetes entwickelt. Diese transgenen Mäuse bieten erstmals die Möglichkeit, die Rolle eines defekten GIP-Rezeptors bei der Entstehung von Diabetes zu klären. Da sich in diesem Mausmodell der Diabetes bereits in den ersten zwei Lebenswochen manifestiert, können neben den langfristigen Auswirkungen auch die Auswirkungen von Diabetes in der juvenilen Phase der Entwicklung untersucht werden. Die transgenen Mäuse stellen darüberhinaus ein in dieser Form einzigartiges Tiermodell bereit, an dem Verfahren entwickelt werden können, die auf eine Therapie von Diabetes abzielen.

Basis für die vorliegende Erfindung ist die Erzeugung transgener Mauslinien, die einen dominant negativen GIP-Rezeptor unter der Kontrolle des Insulin-Promotors in den Beta-Zellen des endokrinen Pankreas exprimieren. Als Folge dieser Expression entwickeln diese transgenen Mäuse in den ersten zwei Lebensmonaten einen schweren Diabetes.

"Dominant" heißt, daß das mutierte Rezeptorprotein in einer so hohen Menge exprimiert wird, daß dem endogenen GIP-Rezeptor kein GIP-Hormon zur Bindung zur Verfügung steht. Dies wird dadurch erreicht, daß der verwendete Insulinpromotor wesentlich stärker ist als der in den Wirtszellen aktive, endogene Promotor. "Negativ" heißt, daß durch die fehlende Signaltransduktion des mutierten GIP-Rezeptors das GIP-Hormon keine Wirkung in den beta-Zellen entfalten kann.

### Gezielte Mutagenese des humanen GIP-Rezeptors und funktioneller Test in Zellkultur

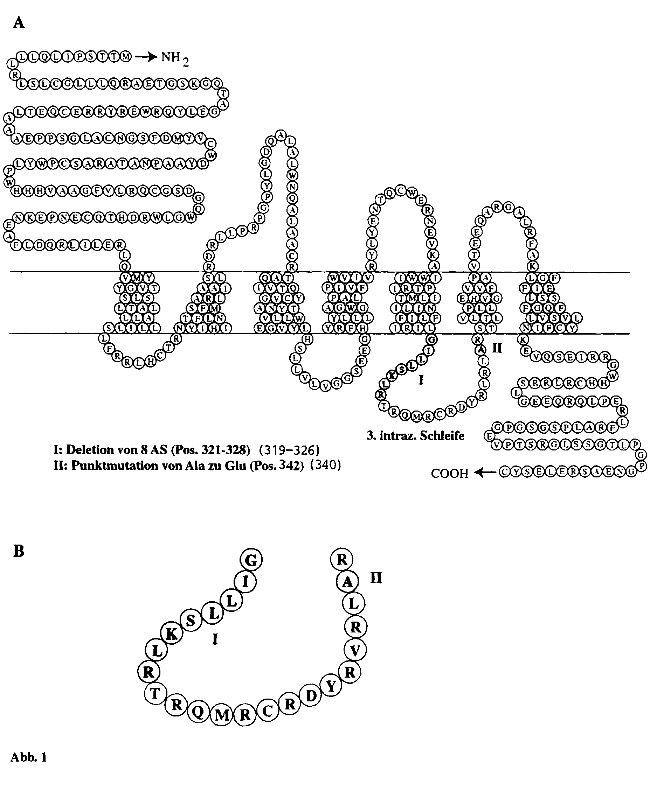

Wir haben durch gezielte Mutagenese einen veränderten GIP-Rezeptor kloniert, der das Peptidhormon GIP bindet, jedoch keine Signaltransduktion einleiten kann. In der dritten intrazellulären Schleife des Rezeptors wurden 8 Aminosäuren (Pos. 321-3-28) deletiert und außerdem eine Aminosäure ausgetauscht (Ala zu Glu an Pos. 342; siehe Abb. 1). In der Familie der Sieben-Transmembran-Rezeptoren ist diese dritte intrazelluläre Schleife, die an der G-Protein-Bindung beteiligt ist, für die Signaltransduktion essentiell (Lefkowitz et al., 1988, Venter et al., 1989).

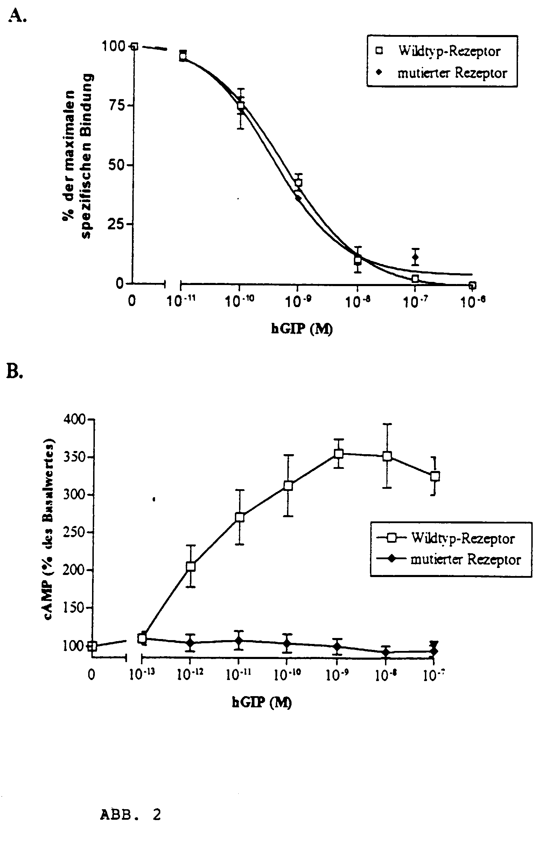

Nach Transfektion in CHL-Zellen zeigt der mutierte GIP-Rezeptor spezifische Bindung von GIP (Kd=0,35 X 10-9 M); er bindet GIP also mit etwa der gleichen Affinität wie der Wildtyp-Rezeptor (Kd=0,22 X 10-9 M, siehe Abb. 2A). Nach Stimulation mit GIP konnte in diesen Zellen keine Erhöhung des cANP-Spiegels gemessen werden (siehe Abb. 2B). Eine starke Überexpression des mutierten GIP-Rezeptors in den Beta-Zellen des endokrinen Pankreas in vivo sollte demnach zu einer Inaktivierung des endogenen GIP-Rezeptors führen, da sämtliches verfügbares GIP-Hormon vom mutierten Rezeptor gebunden wird.

### Expression des funktionell inaktiven GIP-Rezeptor in den Beta-Zellen des endokrinen Pankreas transgener Mäuse

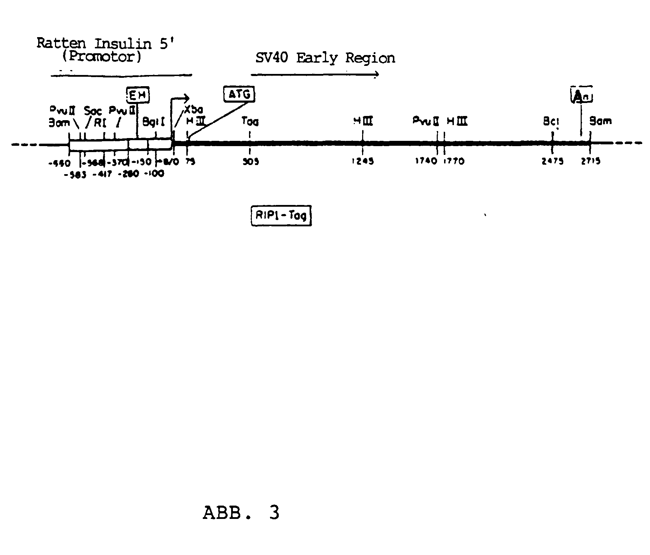

Um eine ß-Zell spezifische Exression des funktionell inaktiven GIP-Rezeptor zu erreichen, wurde die cDNA des mutierten GIP-Rezeptors hinter den Insulinpromotor des RIP1-Vektors (Hanahan, 1985) kloniert (siehe Abb.3). Dieses Konstrukt wurde für die Erzeugung von transgenen Mäusen verwendet. Mit Hilfe des RIP1-Vektors gelang in transgenen Mäusen bereits erfolgreich die Expression verschiedener Gene in den beta-Zellen des endokrinen Pankreas (Hanahan, 1985; Kato et al., 1994).

Transgene Mäuse, die den funktionell inaktiven GIP-Rezeptor in den Beta-Zellen des endokrinen Pankreas exprimieren, wurden durch bekannte Techniken hergestellt. Zunächst wurden 3-4 Monate alte Weibchen superovoliert. Dazu wurde den Tieren 5 I.U. PMSG (0,15 ml pregnant mare's serum gonadotropin pro Maus) in die Bauchhöhle injiziert. 48 Stunden später erhielten die Tiere eine Injektion von 5 I.U. HCG (0,15 ml humanes Gonadotropin pro Maus). Anschließend wurden die Tiere über Nacht verpaart und am nächsten Tag durch cervikale Dislokation getötet. Aus den Eileitern wurden die befruchteten Eizellen isoliert. Unter einem Mikroskop wurde dann das DNA-Konstrukt in einen der Pronuklei befruchteter Eizellen injiziert.

Nach erfolgter Mikroinjektion wurden die befruchteten Eizellen unter Narkose in den Eileiter zyklussynchroner scheinträchtiger Weibchen (Fostermäuse) überführt, die die sich daraus entwickelnden Embryonen austragen und aufziehen. Der Embryotransfer erfolgt unter Narkose (0,5 ml Hypnodil pro Maus). Im narkotisierten Zustand wurde an der Fostermaus auf der Rückseite entsprechend der Lage der Ovarien ein 0,5 cm langer Haut- und Faszienschnitt angebracht, die Ovarien mit den Tuben, bzw. dem Uterus etwas abgehoben und die Präimplantationsembryonen mit einer ausgezogenen Pasteurpipette transferiert. Der Wundverschluß erfolgt mit "Autoclips" (Wundklammern).

Potentiell transgene Tiere wurden im Alter von 3-4 Wochen am Ohr markiert und am Schwanz biopsiert, um die Transgenität zu überprüfen. Transgene Tiere wurden dann mit normalen Mäusen verpaart, um eine Linie zu etablieren. Der Nachwuchs aus diesen Paarungen wird dann ebenfalls im Alter von 3-4 Wochen am Ohr markiert und zur Bestimmung der Transgenität am Schwanz biopsiert.

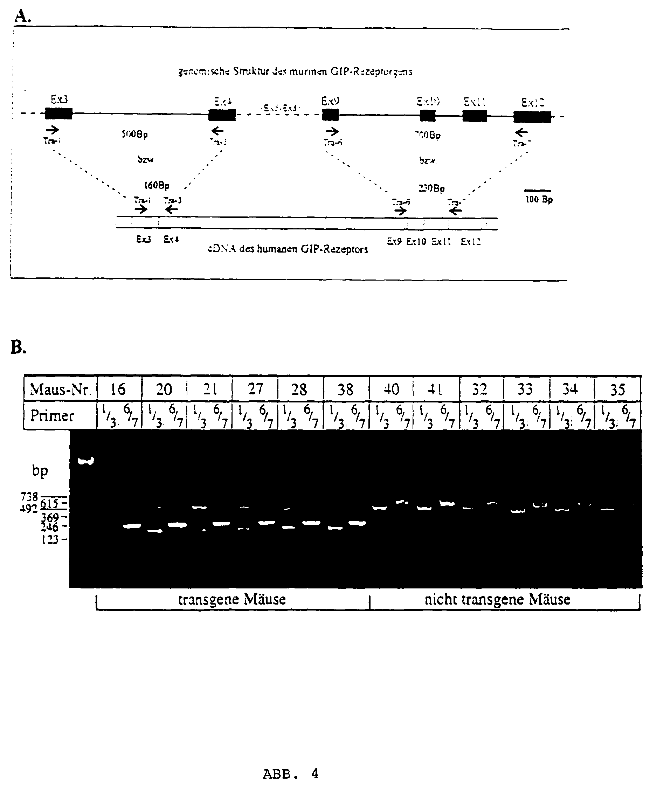

Insgesamt wurden 13 individuelle transgene Linien hergestellt. Die Transgenität wurde durch zwei unabhängige PCR-Analysen überprüft (siehe Abb. 4).

Es ist bekannt, daß ein gegebenes DNA-Expressionskonstrukt in unabhängig erzeugten, transgenen Mauslinien nicht jeweils gleichstark exprimiert wird. Vielmehr ist die Expressionsstärke eines DNA-Abschnittes, der in das Genom integriert ist, sowohl vom Integrationsort als auch von der Anzahl der integrierten DNA-Kopien abhängig. Drei unabhängig etablierte transgene Mauslinien, GIPR^{dn}-A, GIPR^{dn}-B, und GIPR^{dn}-C (Glucose-dependent Insulinotropic Peptide Receptor, dominant negative), die sich durch einen unterschiedlich starken Phänotyp auszeichneten, wurden näher untersucht.

### Bestimmung der Glukosekonzentration im Urin und im Blut

Transgene Männchen der Mauslinien GIPR^{dn}-A, -B, -C wurden mit Wildtyp-Weibchen verpaart. Von den Nachkommen aus diesen Verpaarungen wurde in wöchentlichen Abständen Urin gewonnen und dessen Glukosekonzentration näherungsweise bestimmt. Glukose wird im Urin nachweisbar, wenn die Konzentration im Blut einen kritischen Schwellenwert überschreitet und durch eine Überbeanspruchung der Niere die vollständige Rückresorption nicht mehr gewährleistet ist. Etwa 50 µl Urin wurden auf einen Urinmeßstreifen (Combur10TestÊM, Boehringer Mannheim) aufgetropft und der Meßwert (mg/dl) nach einer Minute abgelesen. Nach Abschluß der Meßreihe wurden die Mäuse zur Feststellung des Genotyps an der Schwanzspitze biopsiert.

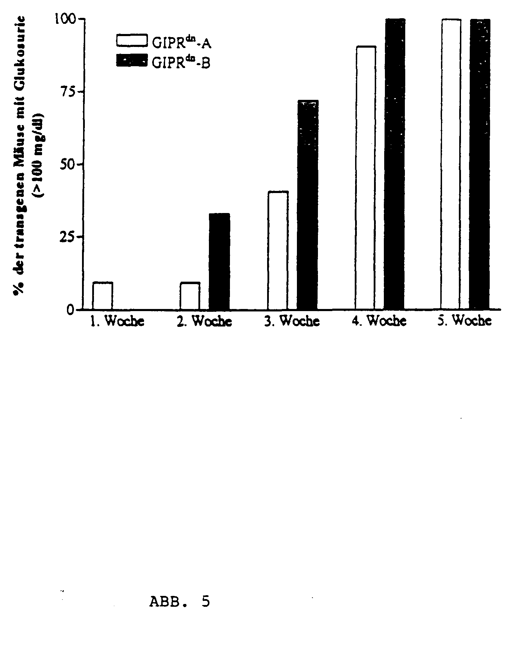

Alle Nachkommen der Mauslinien GIPR^{dn}-A und GIPR^{dn}-B zeigen das Phänomen der Polydipsie (abnorm erhöhte Wasseraufnahme) und entwickeln eine schwere Glukosurie, wobei die 100%ige Penetranz dieses Phänotyp in der Mauslinie GIPR^{dn}-A etwas später eintritt als in der Mauslinie GIPR^{dn}-B (siehe Abb. 5). In der Linie GIPR^{dn}-C konnte dagegen keine Glukosurie festgestellt werden. Interessanterweise kann die Glukosurie in der Linie GIPR^{dn}-B durch die Verabreichung einer kohlenhydratarmen Diät therapiert werden, während dies in der Linie GIPR^{dn}-A nicht möglich ist (ohne Abbildung). Adulte Mäuse der Linien GIPR^{dn}-A und GIPR^{dn}-B zeigten neben der Glukosurie weitere pathologische Auffälligkeiten. Die Obduktion ergab zum einen, daß der Darm abnorm stark gefüllt war und zum anderen, daß Fettkörper weitgehend fehlten.

### Bestimmung der Glukosekonzentration des Blutes (nüchtern und postprandial)

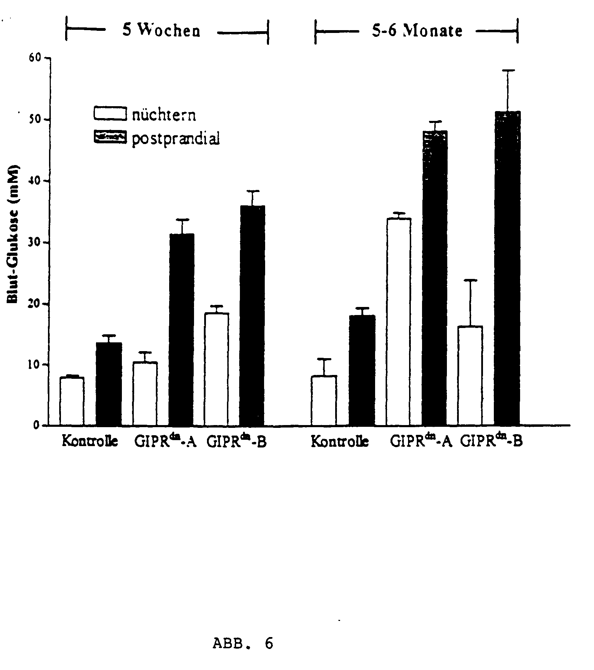

Zur Bestimmung der Glukosekonzentration des Blutes wurden transgene Mäuse (Linie GIPR^{dn}-A und GIPR^{dn}-B) und Kontrollmäuse verschiedenen Alters für 18 Stunden fasten gelassen. Anschließend wurde aus der Schwanzvene 5µl Blut entnommen und die Glukosekonzentration bestimmt (Nüchtern-Wert). Danach wurde den Mäusen für 6 Stunden Futter ad libitum gegeben und anschließend die Glukosekonzentration erneut bestimmt (postprandialer Wert). Diese Untersuchungen zeigen, daß selbst die Nüchternwerte in den transgenen Mäusen bereits 5 Wochen nach der Geburt über denen der Kontrollmäuse liegen (Abb. 6). Entsprechendes gilt für die postprandialen Werte. In den ersten Lebensmonaten verschlechtert sich die Fähigkeit zur Glukoseaufnahme in den transgenen Mäusen weiter. Die nach 5-6 Monaten gewonnene Meßreihe zeigt, daß vor allem die postprandialen Werte gegenüber den zum früheren Zeitpunkt gewonnenen Werten deutlich erhöht sind. Aus diesen Ergebnissen läßt sich schließen, daß sich die Insuffizienz der Glukoseaufnahme in den transgenen Mauslinien GIPR^{dn}-A und GIPR^{dn}-B sukzessive manifestiert.

### Oraler Glukosetoleranztest

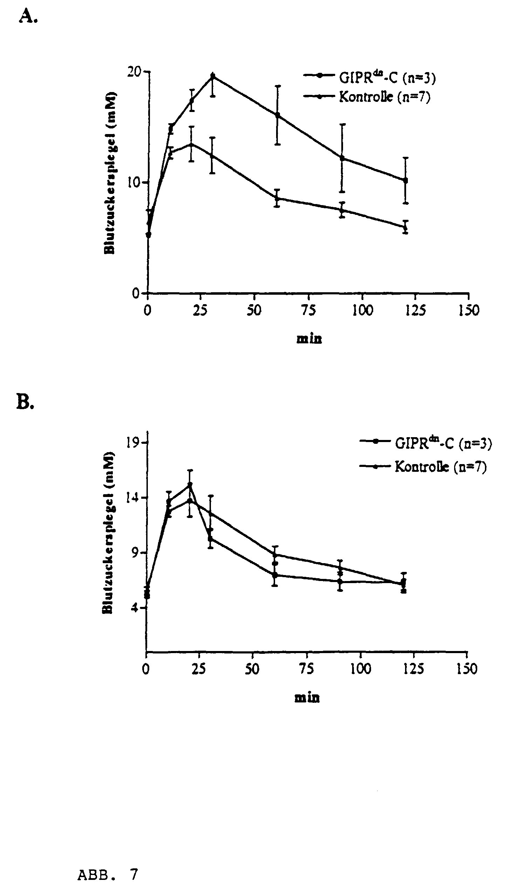

Eine schwache Expression des Transgens kann dazu führen, daß zirkulierendes GIP nicht vollständig vom dominant negativen GIP-Rezeptor, sondern teilweise auch vom endogen exprimierten GIP-Rezeptor gebunden wird. In diesem Fall wäre der endogene GIP-Rezeptor nur teilweise inaktiviert. Um zu überprüfen, ob die transgenen Mäuse der Linie GIPR^{dn}-C, in der keine Glukosurie festgestellt werden konnte, dennoch eine gestörte Fähigkeit zur Glukoseaufnahme besitzen, wurde ein oraler Glukosetoleranztest (Scrocchi et al. 1996) durchgeführt. Dazu wurden adulte Mäuse für 18 Stunden fasten gelassen und anschließend die Glukosekonzentration des Blutes bestimmt. Danach wurde eine lM Glukoselösung (250µl/30g Körpergewicht) per Knopfsonde in den Magen der Versuchstiere gegeben und nachfolgend zu verschiedenen Zeitpunkten die Glukosekonzentration des Blutes bestimmt. Diese Untersuchung ergab den überraschenden Befund, daß männliche Nachkommen der Mauslinie GIPRdn-C eine gestörte Glukosetoleranz zeigten, während die Meßwerte der weiblichen Nachkommen denen der Kontrollen ähnelten (Abb. 7).

### Histopathologische Untersuchungen

Verschiedene Organe aus adulten Mäusen der transgenen Linien GIPRdn-A und GIPRdn-B wurden histopathologisch untersucht. Im Pankreas der transgenen Mäuse ist die Anzahl der Langerhans'schen Inseln deutlich verringert. Darüberhinaus sind die vorhandenen Inseln gegenüber denen von Kontrollmäusen kleiner. Die Lebern der transgenen Mäuse zeigen abnorme Anreicherungen von Glykogen. In den Glumeroli der Nieren konnte die Anwesenheit von prall mit Glykogen gefüllten Zellen - sogenannte Armanni-Ebstein-Zellen nachgewiesen werden. Die pathologischen Befunde bestätigten, daß die transgenen Mauslinien GIPR^{dn}-A und GIPR^{dn}-B eine schwere Form von Diabetes mellitus entwickeln.

### Literatur:

Andrikopoulos, S., Rosella, G., Gaskin, E., Thorburn, A., Kaczmarczyk, S., Zajak, J.D. and Proietto, J. (1993). Impaired regulation of hepatic fructose-1,6-bisphosphatase in the New Zealand obese mouse model of NIDDM. Diabetes 42: 1731-1736
Fehmann, H.C. & Göke, B. (1995). Cell and molecular biology of the incretin hormones glucagon-like-peptide-I and glucose-dependent insulinreleasing polypeptide. Endocrine Reviews 16, 390-410
Gettys, T.W., Ramkumar, V., Surwit, R.S. and Taylor, I.L. (1995). Tissue-specific alterations in G protein expression in genetic versus diet induced models of non-insulin-dependent diabetes mellitus in the mouse. Metabolism 44:771--778
Hanahan, D. (1985). Heritable formation of pancreatic b-cell tumours in transgenic mice expressing recombinant insulin/simian virus 40 oncogenes. Nature 315, 9, 115-122
Kato, I., Suzuki, Y., Akabane, A., Yonekura, H., Tanaka, O., Kondo, H., Takasawa, S., Yoshimoto, T. and Okamoto, H. (1994). Transgenic mice overexpression human vasoactive intestinal peptide (VIP) gene in pancreatic b cells. J. Biol. Chem. 269, 21223-21228
Lefkowitz, R.J. and Caron, M.G. (1988). Adrenergic receptors. Models of the study of receptors coupled to guanine nucleotide regulatory proteins. J. Biol. Chem. 263, 4993-4996
Scrocchi, L.A., Brown, T.J., MacLusky, N., Brubaker, P.L., Auerbach, A.B., Joyner, A.L. and Drucker, D.J. (1996). Glucose intolerance but normal satietyin mice with null mutation in the glucagon-like peptide I receptor gene. Nature Medicine 2, 1254-1258
Roe, M.W., Philipson, L.H., Frangakis, C.J., Kuznetsov, A., Mertz, R.J., Lancaster, M.E., Spencer, B., Worley, J.F. and Dukes, I.D. (1994). Defective glucose dependent endoplasmic reticulum Ca2+ sequestration in diabetic mause islets of Langerhans. J. Biol. Chem. 269:18279-18282
Ueda, H., Ikegami, E., Yamato, E., Fu, J., Fukuda, M., Shen, G., Kawaguchi, Y., Takekawa, K., Fujioka, Y. and Fujisawa, T. (1995). The NSY mouse: a new animal model of spontaneous NIDDM with moderate obesity. Diabetologia 38: 503-508
Venter, J.C., Fraser, C.M., Kerlavage, A.R. and Buck, M.A. (1989). Molecular biology of adrenergic and muscarinergic cholinergic receptors. A perspective. Biochem. Pharmacol. 38, 1197- 1208
Volz, A., Göke, R, Lankat-Buttgereit, B., Fehmann, H.C., Bode, H.P. and Göke, B. (1995). Molecular cloning, functional expression, and signal transduction of the GIP-receptor cloned from a human insulinoma. FEBS Lett. 373, 23-29
Zask, A., Jirkowsky, I., Nowicki, J.W. and McCaleb, M.L. (1990). Synthesis and antihyperglycemic activity of novel 5-(naphtalenylsulfonyl)-2,4-thiazolidinediones. J. Med. Chem. 33: 1418-1423

### Die Abbildungen zeigen:

**Abb.1**
   A. Vereinfachte Darstellung der Aminosäuresequenz des humanen GIP-Rezeptors und dessen Anordnung in der Plasmamembran. Zur Erzeugung eines dominant-negativen GIP-Rezeptors wurden die angegebenen Mutationen in der dritten intrazellulären Schleife des GIP-Rezeptors eingeführt.
   B. Vergrößerte Darstellung der 3. intrazellulären Schleife.
**Abb. 2** Funktionelle Charakterisierung des dominant-negativen GIP-Rezeptors im Vergleich zum normalen, humanen (GIP-Rezeptor. (**A**) Bindung des Liganden (GIP. I¹²⁵-markiertes, humanes GIP(Aminosäuren 1-42) wurde zusammen mit verschiedenen Konzentrationen an unmarkiertem GIP zu stabil transfizierten CHL-Zellen gegeben. Das Maß für die am Rezeptor gebundenen, markierten GIP-Moleküle wurde durch Szintillationsmessung erhalten. Die Bindungsaffinität des mutierten GIP-Rezeptors gleicht der des Wildtyp-Rezeptors. (**B**) Bestimmung der Signaltransduktion des normalen und mutierten GIP-Rezeptors. Zu stabil transfizierten CHL-Zellen wurden unterschiedliche Mengen GIP gegeben. Die radioimmunologische Bestimmung des cAMP-Gehaltes wurde mit Hilfe des "Cyclic AMP Radioimmunoassay-Kits" (Fa. Immunotech., Marseille) durchgeführt. Die erhaltenen Werte zeigen deutlich, daß die Bindung von GIP an den mutierten GIP Rezeptor keine Erhöhung des cAMP-Spiegels in der Zelle erzeugt.
ABB. 3: Struktur des RIP1-Vektors
**Abb. 4** Genotypisierungen von potentiell transgenen Mäusen. (**A**) Lokalisation der für die Genotypisierung verwendeten Oligonukleotidpaare (Tra-1 und Tra-3 bzw Tra-6 und Tra-7) in der genomischen Struktur des murinen GIP-Rezeptorgens bzw. in der mutierten cDNA des humanen GIP-Rezeptors. Die Größen der zu erwartenden DNA-Fragmente sind angegeben. (**B**) Ergebnis der Genotypisierung mittels PCR. Durch die Verwendung von degenerierten Oligonukleotiden wurde aus der genornischen DNA transgener Mäuse (von insgesamt 13 sind exemplarisch Nr. 16, 20, 21, 27, 28, 38 dargestellt) sowohl Intron enthaltende. genomische DNA-Fragmente aus dem GIP-Rezeptorgen der Maus als auch das cDNA-Fragment des humanen GIP-Rezeptors amplifiziert Aus der genomischen DNA nicht-transgener Mäuse (Nr 40, 41, 32, 33, 34, 35) konnte lediglich das Intron enthaltende Fragment des murinen GIP-Rezeptorgens amplifiziert werden Ex. Exon. BP. Basenpaare
**Abb. 5** Entwicklung der Glukosurie in den transgenen Mauslinien *GIPR*^{dn}*-*A und *GIPR*^{dn}*-*B Sämtliche Nachkommen beider Linien entwickeln in den ersten 5 Lebenswochen eine Glukosurie. Bis zu diesem Zeitpunkt ist der Anteil an Mäusen mit Glukosurie in der Linie *GIPR*^{dn}-A etwas geringer als der in der Linie *GIPR*^{dn}-B*.*
**Abb. 6** Erhöhte Glukosekonzentrationen im Blut der transgenen Mauslinien *GIPR*^{dn}-A und *GIPR*^{dn}-B. Sowohl die Nüchternwerte als auch die postprandialen Werte der transgenen Mäuse sind 5 Wochen nach der Geburt deutlich gegenüber denen der Kontrollmäuse erhöht. Eine weitere Verschlechterung der Glukoseaufnahme in den transgenen Mauslinien zeigt sich nach 5-6 Monaten, wo die postprandialen Werte gegenüber denen zum früheren Zeitpunkt gewonnenen Werten deutlich erhöht sind.
**Abb. 7** Gestörte Glukosetoleranz in männlichen Mäusen der transgenen Mauslinie *GIPR*^{dn}-C. Nüchternen Versuchstieren wurde eine lM Glucoselösung (250 ul/30g Körpergewicht) per Knopfsonde verabreicht. (**A**) Die anschließend gemessenen Glukosekonzentrationen im Blut zeigen, daß die Werte bei den männlichen Mäusen gegenüber den Kontrolltieren deutlich erhöht waren. (**B**) Die Glukosekonzentrationen im Blut von weiblichen Mäusen der transgenen Linie *GIPR*^{dn}*-*C zeigten dagegen keine signifikanten Unterschiede im Glukosetoleranztest gegenüber den Kontrolltieren.
ABB. 8 Vereinfachte Darstellung der Aktivierung der Insulinsekretion und der Tranakription des Insulingens durch die Wirkung der Peptidhormone GIP und GLP-l. Die Hormone bewirken über mehrere Zwischenschritte eine Erhöhung der Konzentrationen der second-messenger cAMP und Calcium. Dadurch wird sowohl die Insulinsekretion, als auch die Expression des Insulingens aktiviert.

## Patentansprüche

1. Rekombinante DNA, umfassend einen Promotor, der in den ß-Zellen des endokrinen Pankreas exprimierbar ist, eine Sequenz, die für ein GIP-Rezeptorprotein oder ein aktives Fragment hiervon kodiert, das befähigt ist, das Peptidhormon GIP zu binden, ohne eine Signaltransduktion zu initiieren, eine Terminationssequenz und eine Polyadenylierungssequenz, wobei die DNA bei Expression in einem geeigneten Wirt einen Diabetes-Phänotyp bewirkt.

2. Rekombinante DNA nach Anspruch 1,
dadurch gekennzeichnet,
daß das GIP-Rezeptorprotein oder ein aktives Fragment hiervon in der dritten intrazellulären Schleife, so mutiert ist, daß eine Bindung von GIP an den GIP-Rezeptor möglich ist, ohne eine Signaltransduktion zu initiieren.

3. Rekombinante DNA nach Anspruch 1 oder Anspruch 2,
dadurch gekennzeichnet,
daß das GIP-Rezeptorprotein oder ein aktives Fragment hiervon durch eine oder mehrere Deletionen und/oder Additionen und/oder durch einen oder mehrere Basenaustausche so mutiert ist, daß eine Bindung von GIP möglich ist, ohne eine Signaltransduktion zu initiieren.

4. Rekombinante DNA nach Anspruch 3,
dadurch gekennzeichnet,
daß das GIP-Rezeptorprotein oder ein aktives Fragment hiervon eine Deletion in den Aminosäuren 321-328 (319-326) und einen Aminosäureaustausch in der Position 342 (340) enthält.

5. Rekombinante DNA nach Anspruch 4,
dadurch gekennzeichnet,
daß an der Position 342 Alanin durch Glutaminsäure ausgetauscht wurde.

6. Rekombinante DNA nach einem oder mehreren der vorhergehenden Ansprüche,
dadurch gekennzeichnet,
daß der Promotor ein Insulin-Promotor ist.

7. Rekombinante DNA nach einem oder mehreren der vorhergehenden Ansprüche,
dadurch gekennzeichnet,
daß die für das GIP-Rezeptorprotein kodierende Sequenz oder ein aktives Fragment dieser Sequenz genomische DNA oder cDNA ist.

8. Rekombinante DNA nach einem oder mehreren der vorhergehenden Ansprüche,
dadurch gekennzeichnet,
daß die rekombinante DNA den RIP1-Vektor umfaßt, enthaltend, in funktioneller Verbindung, eine GIP-Rezeptorprotein-Sequenz oder ein aktives Fragment hiervon, die so verändert ist, daß das Peptidhormon GIP an den GIP-Rezeptor bindet, ohne eine Signaltransduktion zu initiieren.

9. Rekombinante DNA nach einem oder mehreren der vorhergehenden Ansprüche,
dadurch gekennzeichnet,
daß die für das GIP-Rezeptorprotein kodierenden Sequenzen oder aktive Fragmente hiervon in Säugerzellen exprimierbar sind.

10. Vektor, umfassend eine rekombinante DNA nach einem oder mehreren der vorhergehenden Ansprüche.

11. Eukaryontische oder prokaryontische Zellinie, enthaltend eine rekombinante DNA oder einen Vektor nach einem oder mehreren der vorhergehenden Ansprüche.

12. Rekombinantes GIP-Rezeptorprotein oder aktives Fragment hiervon und hierfür kodierende DNA,
dadurch gekennzeichnet,
daß das GIP-Rezeptorprotein oder ein aktives Fragment hiervon oder die hierfür kodierende DNA so verändert wurde, daß das GIP-Peptidhormon bindet, ohne eine Signaltransduktion zu initiieren.

13. Transgenes, nicht menschliches Säugetier, enthaltend eine rekombinante DNA nach einem oder mehreren der vorhergehenden Ansprüche.

14. Transgenes, nicht menschliches Säugetier nach Anspruch 13, dadurch gekennzeichnet, daß das Tier ein Nager ist.

15. Transgenes, nicht menschliches Säugetier nach Anspruch 14, dadurch gekennzeichnet, daß der Nager eine Maus oder eine Ratte ist.

16. Verfahren zur Herstellung eines transgenen, nicht menschlichen Säugetiers nach einem oder mehreren der Ansprüche 13 bis 15,
dadurch gekennzeichnet,
daß eine rekombinante DNA nach einem oder mehreren der Ansprüche 1 bis 9 in ein nicht menschliches Säugetier in der Weise eingeführt wird, daß ein transgenes, nicht menschliches Säugetier mit der Fähigkeit zur Entwicklung eines Diabetes-Phänotyps entsteht.

17. Verwendung eines transgenen, nicht menschlichen Säugetiers nach einem oder mehreren der vorhergehenden Ansprüche als Modellsystem zur Untersuchung der Entstehung, Therapie und Spätfolgen von Diabetes mellitus.
